(19) Europäisches Patentamt
European Patent Office
Office européen des brevets

(11) **EP 4 182 650 B1**

(12) **EUROPEAN PATENT SPECIFICATION**

(45) Date of publication and mention
of the grant of the patent:
**29.04.2026 Bulletin 2026/18**

(21) Application number: **21752752.2**

(22) Date of filing: **16.07.2021**

(51) International Patent Classification (IPC):
*A61B 5/00* (2006.01)    *G16H 50/30* (2018.01)
*G01J 5/00* (2022.01)    *A61B 5/01* (2006.01)
*G01J 5/06* (2022.01)

(52) Cooperative Patent Classification (CPC):
**G01J 5/0025; A61B 5/0077; A61B 5/01;
A61B 5/7203; A61B 5/7246; A61B 5/7264;
G01J 5/06; G16H 50/30;** A61B 2560/0252;
G01J 2005/0077

(86) International application number:
**PCT/IB2021/056423**

(87) International publication number:
**WO 2022/013822 (20.01.2022 Gazette 2022/03)**

(54) **FEVER DETECTOR BY DISTANT MULTIPIXEL THERMAL IMAGING**

FIEBERDETEKTOR DURCH ENTFERNTE MULTIPIXEL-WÄRMEBILDGEBUNG

DÉTECTEUR DE FIÈVRE PAR IMAGERIE THERMIQUE MULTIPIXEL À DISTANCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.07.2020 US 202063052936 P**

(43) Date of publication of application:
**24.05.2023 Bulletin 2023/21**

(73) Proprietor: **Meridian Innovation Ltd, Hong Kong
Hong Kong (HK)**

(72) Inventors:
• **MARKOV, Stanislav Nikolaev
Hong Kong (CN)**

• **LAU, Yat Fei
Hong Kong Science Park Pak Shek Kok, N. T.,
Hong
Kong (CN)**

(74) Representative: **Kompter, Hans-Michael
Heumann
Rechtsanwälte- und Patentanwälte
Spessartring 63
64287 Darmstadt (DE)**

(56) References cited:
**CN-A- 110 196 103    IT-A1- MI20 100 102
US-A1- 2008 154 138**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present disclosure relates generally to non-contact temperature scanning using a thermal camera.

BACKGROUND

The occurrence of pandemics, such as SARS, MERS, EBOLA and COVID-19, along with the inevitable need to manage and contain the spread of a disease on a large scale, has triggered a widespread demand for non-contact measurement of human temperature in the context of mass screening for fever.

Current approaches for non-contact measurement of human temperature include the use of forehead infrared thermometers, and distant thermal imaging cameras operating in the long-wave infrared (LWIR) spectrum. In both approaches, the devices measure only the surface temperature of a subject's skin, not the core body temperature. Both approaches are subject to inherent inaccuracies. For example, in the case of forehead infrared thermometers, the actual oral temperature of a subject, even based on established reference values and calculations, the actual oral temperature could fall below 35°C. For the case of a distant thermal imaging camera, inaccuracies can occur due to the difference in the ambient temperature of the environment of the setup of the device and the ambient temperature of the environment from where the subject came unless the subject's temperature reaches a steady-state prior to measuring. As such, a wait time is required before a subject can be measured in order for an accurate reading. This, however, is inconvenient.

The US patent application US 2008/154138 A1 discloses a temperature scanning system and method. The Chinese patent application CN 110 196 103 A teaches a temperature measuring method, using an infrared camera, for determining if the temperature of a person is within a target temperature interval. However, none of these documents teaches or suggests a human mask generator and a hot spot generator.

The present disclosure is directed to a thermal imaging device for fast and accurate mass fever or temperature screening.

SUMMARY

Embodiments of the present disclosure generally relate to devices and methods for fast and accurate non-contact temperature screening.

In one embodiment, a temperature scanning system is provided comprising:

a thermal image capture module configured to capture thermal images; a storage unit; an ambient sensor module configured to capture ambient conditions including ambient temperature (TA); and
a processing module configured to process a thermal image, the processing module comprises

an image processing unit, the image processing unit processes the thermal image and includes
a human mask generator configured to generate a human mask which extracts a first set of pixels representing the subject from the thermal image, and
a hot spot generator configured to generate a hot spot mask which extracts a second set of pixels from the first set of pixels representing a forehead of the subject in the thermal image to detect a hot spot of the human subject in the thermal image; and
a temperature calculation unit, the temperature calculation unit is configured to estimate a skin temperature (Ts) of a subject in the thermal image, and

calculate a core temperature (Tc) of the subject based on an arithmetic mean value or a median value of the pixels within the hot spot mask and current ambient conditions, using a biophysical model, wherein the biophysical model is defined as $TC = TS + (TS - TA) h\lambda / k$,
wherein

TC is the core temperature,
TS is the skin temperature as estimated by the temperature calculation unit,
TA is the current ambient temperature as determined by the ambient temperature sensor module,
h = the convection coefficient between the forehead skin and the ambient air,
$\lambda$ = a constant which is equal to a characteristic length,
k = the effective thermal conductivity of layers between the brain from the outer surface of the forehead skin, and
$h\lambda / k$ = the parametric factor which defines the relationship between TC, TS and TA;

and the parameter being stored in the storage unit of the system. In another embodiment, the invention relates to a method for non-contact temperature scanning comprising:

capturing a thermal image of a subject with current ambient conditions; processing the thermal image to detect a hot spot of the subject, wherein the processing comprises
generating a subject mask for the thermal image by extracting a first set of pixels representing the subject,
generating a hot spot mask for the hot spot on the subject mask by extracting a second set of pixels from the first set of pixels, and
determining a skin temperature TS of the subject from the hot spot on the subject mask; and
calculating a core temperature Tc of the subject based on an arithmetic mean value or a median value of the pixels within the hot spot mask taking account of the current ambient conditions, which includes an ambient temperature (TA) using a biophysical model, the biophysical model (260) is defined as $TC = TS + (TS - TA) h\lambda / k$, where

TC is the core temperature,
TS is the skin temperature as estimated by the temperature calculation unit,
TA is the current ambient temperature,
h = the convection coefficient between the forehead skin and the ambient air,
$\lambda$ = a constant which is equal to a characteristic length,
k = the effective thermal conductivity of layers between the brain from the outer surface of the forehead skin, and
$h\lambda / k$ = the parametric factor which defines the relationship between TC, TS and TA, and storing the parameter.

These and other advantages and features of the embodiments herein disclosed, will become apparent through reference to the following description and the accompanying drawings. Furthermore, it is to be understood that the features of the various embodiments described herein are not mutually exclusive and can exist in various combinations and permutations.

BRIEF DESCRIPTION OF THE DRAWINGS

The accompanying drawings, which are incorporated in and form part of the specification, illustrate preferred embodiments of the present disclosure and, together with the description, serve to explain the principles of various embodiments of the present disclosure.

Fig. 1 shows a simplified embodiment of a thermal imaging system for fast and accurate temperature screening;
Fig. 2 shows a simplified process for fast and accurate temperature screening;
Fig. 3a depicts an embodiment of a set-up process;
Fig. 3b is a simplified depiction of an image processing process of a thermal imaging system;
Fig. 4 shows an embodiment of a system for temperature screening;
Fig. 5 shows an embodiment of a temperature screening process; and
Fig. 6 shows a process for obtaining parameters of an empirical model.

DETAILED DESCRIPTION

Embodiments relate to systems and methods for fast and accurate temperature screening. The systems and methods enable non-contact temperature screening of subjects to determine core body temperature fast and accurately. To increase accuracy, determining the core body temperature requires ambient conditions to be taken into account, particularly those which affect the measured skin temperature of a subject.

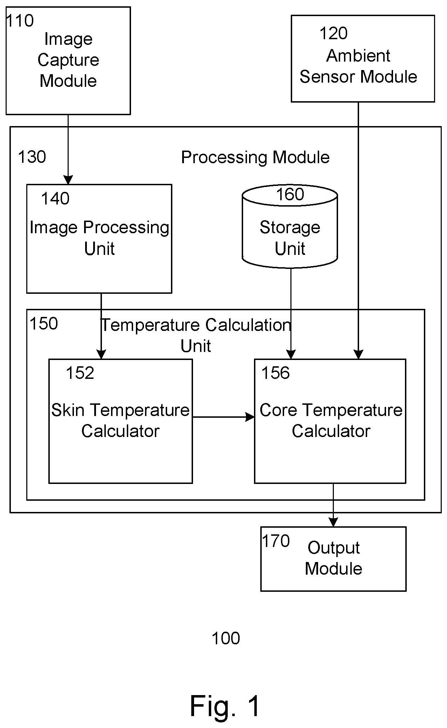

Fig. 1 shows a simplified block diagram of an embodiment of a temperature screening system 100. As shown, the system includes an image capture module 110, an ambient sensor module 120, a processing module 130 and an output module 170.

In one embodiment, the image capture module 110 includes a thermal imaging camera configured to capture thermal images. The thermal imaging camera, for example, is a high-resolution multi-pixel thermal imaging camera. For example, the thermal imaging camera may be MI0801 imaging camera from Meridian Innovation which is based on CMOS long-wave infrared (LWIR) technology. Other types of thermal imaging cameras may also be useful.

The image capture module can be configured to continuously capture images. The image capture rate, for example, may be about 5 to 30 frames/sec. Other image capture rates may also be useful. In continuous capture mode, the image capture module may capture images without any human subject. The image capture module may also be configured to operate in other modes. For example, the image capture module may be configured to operate in automatic mode when it only senses a human subject or in manual mode controlled by an operator. Other modes of operations may also be provided for the image capture module.

As for the ambient sensor module, it is configured to capture the ambient conditions of the camera module, which is representative of the ambient conditions of a human subject whose temperature is being measured. In one embodiment, the ambient sensor module is configured to captured interior ambient conditions. For example, the ambient sensor module includes sensors configured to capture the local temperature and humidity in the area where and when the subject's image is captured. Providing an ambient sensor module which captures both interior and exterior ambient conditions may also be useful.

In one embodiment, the ambient sensor module includes a temperature sensor and a humidity sensor for sensing the ambient temperature $T_A$ and the ambient relative humidity $R_h$. These may correlate to interior sensors for sensing the ambient conditions of the interior where the image is being captured. The ambient sensor module may include other sensors for measuring other ambient conditions. For example, the sensors may measure the temperature of the subject's clothes and exterior temperature, humidity and air velocity. Other types of sensors may also be included for the ambient sensor module. In one embodiment, the temperature of the clothes and the distribution of the temperature in the building can be estimated from the thermal image itself through image processing. The process is similar to the process of obtaining the estimate of the forehead skin temperature. As for external parameters, such as air velocity, temperature and relative humidity, they may be measured by sensors or acquired from external sources, such as meteorological services or databases.

The processing module 130 includes an image processing unit 140, a temperature calculation unit 150 and a storage unit 160. The processing module is configured to process images captured by the image capture module to determine the core temperatures of subjects, taking current ambient conditions into account. By current ambient conditions, they refer to the ambient conditions at the time the image was taken with an account for the historical trends of the measured values preceding the specific measurement of the given subject. For example, ambient conditions are measured simultaneously with image capturing. The images, for example, are continuously captured and processed one at a time. In some embodiments, to produce more stable results, an averaging across multiple frames of a pipeline may be employed. This averaging refers to both the ambient temperature and humidity values, as well as the processing of the thermal images, and the output of the processing pipeline.

The image processing unit 140 processes an image to detect hot spots. In one embodiment, the image processing unit processes an image in two stages or steps. The first stage includes identifying a human subject from the image. The first stage includes generating a mask corresponding to a human subject in the image. For example, only pixels within the mask are considered. The next stage is to identify a hot region within the mask. The hot region generally corresponds to the forehead of the human subject.

The hot region is processed by the temperature calculation unit 150 to determine a core body temperature Tc of the subject. In one embodiment, determining the core body temperature involves a two-step process. As shown, the temperature calculation unit includes a skin temperature calculator 152 and a core temperature calculator 156. The skin temperature calculator determines a skin temperature of the subject according to the hot region. In one embodiment, the skin temperature calculator calculates a single representative skin temperature $T_S$ for the hot region that was detected. $T_S$ correlates to, for example, a forehead skin temperature. $T_S$ can be used as a proxy to the core body temperature. The skin temperature calculator takes $T_S$ and determines a core body temperature Tc using an empirical biophysical model. The biophysical model is employed to infer core body temperatures based on calculated skin temperature and the heat transfer properties of the human subject, accounting for the ambient conditions, such as the ambient temperature and humidity, which affect the heat transfer processes. For example, the biophysical model correlates the core body temperature Tc to the skin temperature $T_S$ based on ambient conditions or parameters, such as $T_A$ and $R_h$. The coefficients for the empirical biophysical model, for example, are stored in the storage unit 160. The storage unit may store other information required for calculating the core body temperature.

Once the core body temperature Tc is calculated, it is passed to the output module 170. The output module is configured to inform the subject's core body temperature. For example, the core body temperature Tc of the subject is displayed on a monitor (not shown). Should core body temperature Tc exceed a threshold, such as indicating a fever, an alarm may be set. The alarm may be a visual alarm, an audio alarm or a combination thereof. Other types of alarms may also be useful. In some embodiments, the system is tied to a gate for allowing or blocking a subject from passing therethrough.

In some cases, an image may include more than one human subject. The mask, in such cases, corresponds to the human subjects. For example, masks for the human subjects may be obtained within sub-regions of the entire thermal frame and coordinates of the sub-regions may be set using a user interface by an operator or automatically by facial detection using, for example, a visual camera connected to the system. The multiple human subjects in an image may be collectively referred to as a subject or a human subject. The hot regions within the masks are determined. For example, the hot region of each human subject in the image is determined and is used to determine his/her $T_S$ and Tc. In one embodiment, the hot regions are processed one at a time. Other techniques for processing the hot regions may also be useful. For example, depending on the architecture and capabilities of the image processing unit, the hot regions may be processed in parallel.

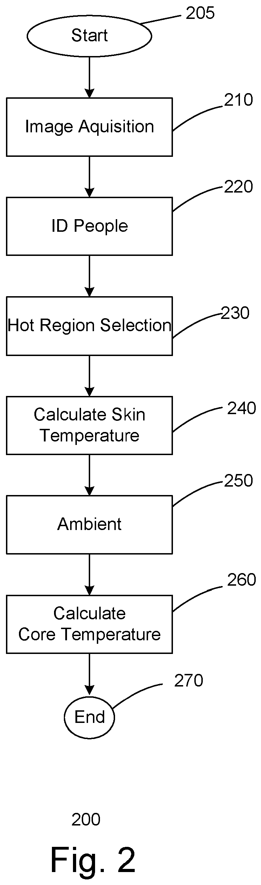

Fig. 2 shows a simplified process 200 for fast and accurate temperature screening. As shown, the process is initiated or started at 205. Starting the process, for example, starts the system to capture images. At 210, images are captured or acquired. For example, the image capture module may be configured to capture images continuously. Configuring the image capture module to capture images in other modes, such as in the automatic mode or the manual mode may also be useful. At 220, an image is processed to identify human subjects. For example, the image processing unit generates a mask which segments a human subject from the image. It is understood that the image may include more than one human subject and the mask segments the human subjects from the image.

The process continues to 230 for determining a hot spot or region of the human subject. In one embodiment, the image processing unit determines the hottest region within the mask. At 240, the skin temperature $T_S$ of the human subject is calculated based on the hot region. In one embodiment, the skin temperature calculator of the temperature calculation unit calculates $T_S$.

At 250, ambient conditions are measured. The ambient conditions, for example, are measured simultaneously with the capturing of the image which is being processed. In one embodiment, the ambient conditions are measured by the ambient sensor module and include ambient temperature $T_A$ and relative humidity $R_h$. Other ambient conditions may also be measured by the ambient sensor module, such as exterior temperature and humidity, air velocity as well as the temperature of the subject's clothes. The $T_S$ and ambient conditions $T_A$ and $R_h$ are employed to determine a core body temperature Tc of the subject using an empirical biophysical model at 260. For example, the core temperature calculator of the temperature calculation unit determines Tc. Parameters or coefficients needed for the biophysical model may be embedded into the system. For example, the parameters may be coded into the software used to operate the system. Alternatively, the parameters may be retrieved from the storage unit or provided through connectivity interfaces of the system. The system may be configured to be continuously updated based on a centralized system that upgrades the biophysical model when appropriate or needed.

Once the core body temperature Tc is calculated, it is provided to a user at 270 which completes the process for determining the Tc of the human subject from the image. For example, an output module is configured to inform the user of the subject's core body temperature Tc. The core body temperature Tc of the subject may be displayed on a monitor. Should Tc exceed a threshold, such as indicating a fever, an alarm may be set. The alarm may be a visual alarm, an audio alarm or a combination thereof. Other types of alarms may also be useful. In this case, the system is tied to a gate for allowing or blocking a subject from passing therethrough.

The process described is performed for each image captured. In the case where an image includes multiple human subjects, Tc is calculated for each human subject.

Typically, the sensors are pre-calibrated by the manufacturer. For example, the sensors are per pixel calibrated during manufacturing. However, there is a finite tolerance which depends on the specific system that embeds the sensors. To reduce this tolerance, which may be affected by various factors, including but not limited to ambient conditions, packaging, electronics that surround the sensors, power supply as well as others, a set-up process may be perfomed for each system.

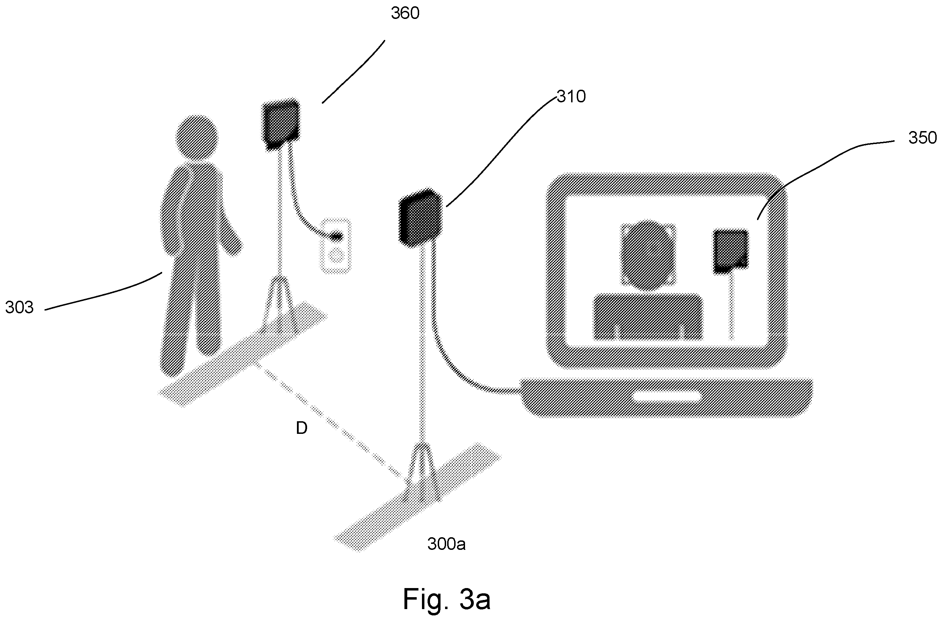

Fig. 3a depicts an embodiment of a set-up process 300a for a temperature screening system. As shown, a thermal imaging camera 310 is set up to capture a thermal image of a human subject 303 at a distance D. A heat source 360 is located at the proximity of the human subject. The heat source is configured to generate a known temperature with a tolerance of at least a few times lower than that of the readout of the thermal camera on a display 350. A comparison between the readout from the pixels viewing the reference heat source and the value of the set temperature of the reference heat source enables a correction offset to be established for reducing the readout error from the pixels viewing the subject whose $T_S$ is estimated. This process can be done continuously for every frame, where the calculation of the correction offset, and the corresponding compensation of the output from the thermal imaging camera can be done simultaneously.

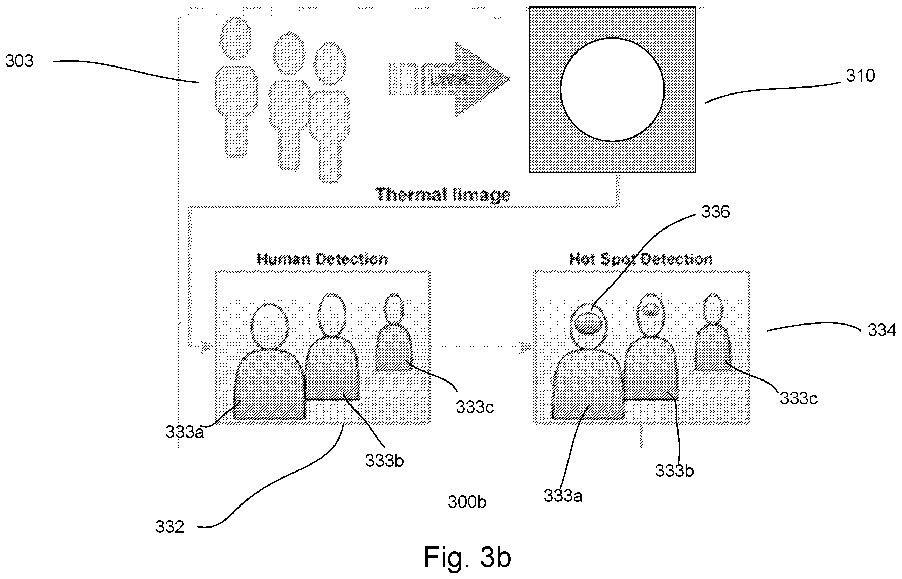

Fig. 3b depicts a simplified embodiment of an image processing process 300b. An image capture module 310 is configured to capture thermal images of, for example, human subjects 303. In one embodiment, the image capture module includes a thermal imaging camera configured to capture thermal images. The thermal imaging camera, for example, is a high-resolution multi-pixel thermal imaging camera, such as a CMOS based LWIR thermal imaging camera. The thermal imaging camera, for example, is capable of capturing images with a resolution of about 1k for close range, such as from 10 to 100 cm and 5k for medium range, such as from 30 cm to 2m. Other resolutions may also be useful. In the case of longer distances, an appropriately narrowed field of view (FOV) may be employed. Other types of thermal imaging cameras may also be useful.

The image capture module may be configured to capture in a continuous capture mode. For example, images are continuously captured at a defined capture rate. The defined capture rate may be the capture rate of the thermal imaging camera of the image capture module. The capture rate, for example, may be about 5 to 30 frames/s. The frame rate may be varied or based on the dynamics of the scene and the noise performance of the thermal imaging sensor and processing capabilities of the host. As shown, 3 human subjects 303 are approaching the image capture module. The human subjects are located at different distances from the thermal imaging camera. For example, in the case of a mass screening, people are walking towards the image capture module, but are at different distances and may approach at different rates. The image capture module may be configured to operate in other modes, such as in automatic and manual modes.

A thermal image 332 with the human subjects is captured by the image capture module and processed by the image processing unit of the processing module. As shown, the thermal image includes 3 human subjects 333a-c at different distances. For example, a first human subject 333a is closest to the image capture module, a second human subject 333b is the next closest to the image capture module and a third human subject 333c is the most distant from the image capture module.

The image processing unit processes a thermal image in two stages or steps. In the first stage, the image processing unit detects human subjects in the thermal image. In one embodiment, a mask is generated which corresponds to the human subjects. For example, only pixels of the thermal image within a mask area are considered while pixels outside of the mask area are ignored. The masked image is then subjected to the second stage of processing. In the second stage, the masked image is processed to detect hot regions or spots 336 in the human subjects. For example, a processed masked image 334 includes hot spots 336 detected in the masked region corresponding to the human subjects. As shown, the first and second human subjects 333a-b each have a hot spot while the third human subject 33c does not. This may be because the third human subject is too far away from the thermal imaging camera range for hot spot detection or to reliably estimate skin temperature. For example, this may depend on various factors, such as FPS and sensitivity of the sensors, lens properties, distance, resolution and FOV. As previously discussed, the hot spots are used to determine $T_S$ by the skin temperature calculator of the temperature calculation unit and Tc by the core temperature calculator of the temperature calculation unit based on $T_S$ and ambient conditions, such as $T_A$ and $R_h$.

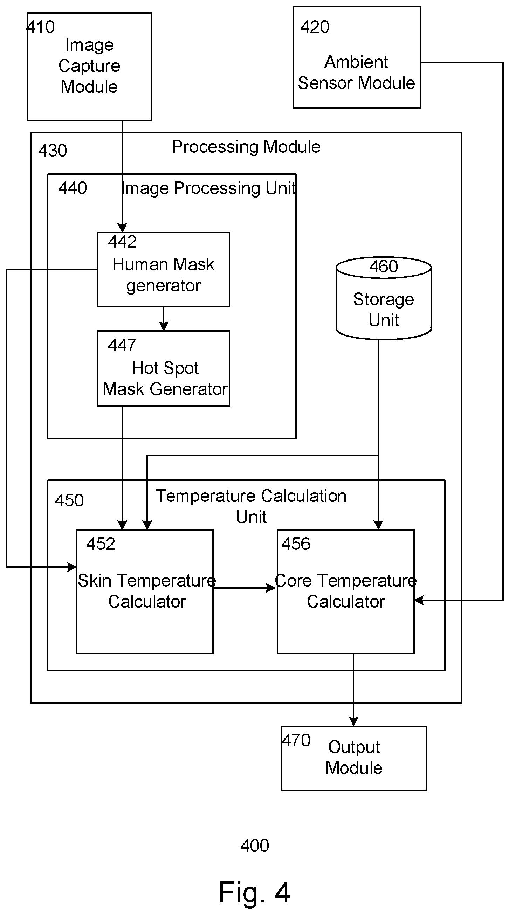

Fig. 4 shows a simplified block diagram of another embodiment of a temperature screening system 400. As shown, the system includes an image capture module 410, an ambient sensor module 420, a processing module 430 and an output module 470.

In one embodiment, the image capture module 410 includes a thermal imaging camera. In one embodiment, the thermal imaging camera is a high-resolution multi-pixel thermal imaging camera, such as a CMOS-based long-wave infrared (LWIR) imaging camera. The thermal imaging camera, for example, is capable of capturing images having a resolution of about 5k. Other image resolutions may also be useful. Furthermore, the thermal imaging camera may be configured to operate in various image capture modes, such as continuous, automatic and manual image capture modes. For example, the imaging camera may be an MI0801 imaging camera from Meridian Innovation. Other types of thermal imaging cameras may also be useful.

The ambient sensor module 420 includes sensors for capturing ambient conditions. For example, the ambient sensor module includes sensors which capture ambient conditions of an image capture area. The ambient conditions of the image capture area, for example, may be referred to as interior ambient conditions. The ambient sensors, in one embodiment, include temperature and humidity sensors to capture the ambient temperature $T_A$ and relative humidity $R_h$. The ambient sensor module may include other sensors for measuring other ambient conditions, such as air velocity. Alternatively, interior air velocity may be obtained from data connectivity to a centralized HVAC system where the camera is placed.

In one embodiment, the system is configured to also capture exterior ambient conditions, such as exterior temperature, humidity and air velocity, from where the human subjects came. The exterior ambient conditions may be obtained by external sensor modules. Other techniques for obtaining exterior ambient conditions may also be useful. For example, external weather conditions, such as temperature and humidity, may be acquired via queries to a local weather observatory. By providing the location of the camera installation, historical and current data of the local location can be obtained.

The processing module includes an image processing unit 440, a temperature calculation unit 450 and a storage unit 460. The processing module is configured to process thermal images captured by the image capture module to determine the core temperatures of subjects, taking current ambient conditions into account.

The image processing unit processes a thermal image to detect hot spots. In one embodiment, the image processing unit includes a human mask generator 442 and a hot spot mask generator 447 for processing a thermal image in a two-stage segmentation process. In the first stage, the human mask generator generates a human mask to segment the human subject or subjects from the thermal image. The human mask, for example, is employed to extract a set of pixels representing a human subject from the thermal image. In one embodiment, the human mask generator employs adaptive thresholding to segment the region corresponding to the human subject as a hot-on-cold contour. The use of adaptive thresholding enables human subject segmentation to be relatively independent of the background, clothing and distance from the thermal imaging camera. After the human mask is generated, the second stage includes generating a hot spot mask by the hot spot mask generator. The hot spot mask segments the hot spots from the human subjects within the human mask.

In one embodiment, the human mask generator includes various components for image enhancement, image normalization and mask generation. For example, the human mask generator may include an image denoiser, an image normalizer, an image blurer, an image renormalizer, and an image mask generator. Providing the human mask generator with other components may also be useful.

The image denoiser enhances the thermal image by removing noise. For example, noise is removed by smoothening out the pixels. The image denoiser, for example, applies filtering in the temporal and spatial domains. In one embodiment, the image denoiser may be an AI image denoiser. For example, the image denoiser is a filter in the spatial domain based on a convolutional neural network for removing noise to enhance the thermal image without losing relevant information and resolution. Other types of image denoisers may also be useful. The output of the image denoiser, for example, is a denoised image.

The denoised image is processed by the image normalizer. The thermal image is an indexed image. Each pixel of the thermal image represents a temperature value. The temperature value is represented by a floating point number. The intensity of a pixel depends on the temperature detected. In one embodiment, the image normalizer normalizes the denoised image by mapping the temperature values of the denoised image to integer values. For example, an integer value is assigned or mapped to a temperature value for each pixel. The range of integer values, in one embodiment, is from 0 - 255 (256 integers for an 8-bit implementation). Other numbers of integer values may also be useful. The image normalizer generates a normalized image.

The normalized image is processed by the image blurer. In one embodiment, the image blurer blurs or smoothens out the interface of the human subject in order to facilitate further processing. For example, an edge of the human subject is smoothened out. Smoothening out the normalized image, for example, makes the temperature transition at the interface smooth rather than abrupt. For example, the image blurer may be a low pass or median filter for removing outlier pixels at the interface of the human subject. The output of the image blurer may be a blurred-normalized image.

The blurred-normalized image is processed by the image mask generator 442 to generate a human mask for the thermal image. In one embodiment, the image mask generator employs adaptive thresholding on the blurred-normalized image to generate the human mask. For example, the image mask generator retrieves parameters for adaptive thresholding from the storage unit and the parameters are used to generate the human mask. Alternatively, the parameters may be embedded into the system. In one embodiment, the parameters for adaptive thresholding include a block size and a constant offset. In one embodiment, the block size is 97 and the constant offset is 29. Other values for the adaptive thresholding parameters may also be useful.

The parameter values, in one embodiment, are empirically obtained. In one embodiment, the parameter values may vary based on the overall properties of a thermal image histogram, which correlate to the background or ambient temperature. The thermal image histogram associates a set of temperature ranges with the number of times a pixel readout falls within any of these temperature ranges. In that sense, they are like the intensity values in grayscale images or like ordinary data histograms. The image mask generator generates a human mask as its output for segmenting the human subject from the thermal image.

The blurred-normalized image may be processed by the image renormalizer. For example, image processing techniques may operate on unsigned integers, such as adaptive thresholding, to increase processing efficiency. In some embodiments, the results are converted to temperature by renormalizing using the image renormalizer. Renormalizing preserves the mapping of temperature to unsigned integer and back. The image renormalizer generates as its output a renormalized-blurred image. In some embodiments, renormalization is not necessary. For example, the calculation of the skin temperature Ts is performed directly based on the pixels of the denoised image and not on the mask itself. The mask merely serves to define the set of pixels that are taken into account during the calculation of Ts. However, blurring helps to smoothen the contours of the mask to improve the reliability of the temperature readouts.

The hot spot mask generator 447 processes the human mask from the human mask generator 442 to generate a hot spot mask. For example, the human mask derived from the blurred-normalized image is processed by the hot spot mask generator. The hot spot mask generator extracts a second set of pixels from the first set of pixels. The second set of pixels corresponds to a hot spot in the human mask. The hot spot generally corresponds to or represents the forehead of the human subject or an equally hot region that may be used as a proxy to the core body temperature of the human subject.

In one embodiment, the hot spot mask generator employs simple thresholding to generate the hot spot mask. The simple thresholding is used to segment a representative region within the human mask. The simple thresholding provides a hot spot mask that correlates well with the forehead region of the subject. This allows us to obtain a single value estimate of the forehead skin temperature based on the statistical average within the hot spot. For example, the arithmetic mean or the median value of the pixels within the hot spot mask correlates strongly or most strongly with the core body temperature.

In one embodiment, the threshold for establishing the hot spot mask is based on the normalized thermal image. For an 8-bit implementation with 256 (0 - 255) integer values, the threshold is in the range of 235 - 245 in the case where the image is normalized such that the minimum temperature is mapped to 0 while the maximum temperature is mapped to 255. For example, pixels with integer values of a minimum of 235 - 245 (based on 0 - 255) form the segmented hot spot region of the hot spot mask. Other threshold values or ranges may also be useful. The threshold value may depend on, for example, the size (number of pixels) of the human mask. The threshold values may be predetermined and programmed into the system. In addition, the predetermined value may be overridden by an operator. For example, an operator may determine the threshold value onsite during setup. The system may also provide adaptively controlled thresholding based on ambient conditions and/or the size of the human mask or hot spot mask. Other implementations of the hot spot mask generator may also be useful. The hot spot mask generator generates a hot spot mask image which is a segmentation of the human mask.

The output of the image processing module is passed to the temperature calculation unit 450 for processing to determine the core body temperature $T_C$ of the human subject. In one embodiment, determining the core body temperature involves a two step process. As shown, the temperature calculation unit includes a skin temperature calculator 452 and a core temperature calculator 456.

The skin temperature calculator 452 determines the skin temperature of the subject according to the hot spot or region. In one embodiment, the skin temperature calculator calculates a single representative skin temperature $T_S$ for the hot region that was detected. In one embodiment, $T_S$ is calculated using a statistical model over the hot spot temperatures. The statistical model is based on the outputs of the image processing module. In one embodiment, the statistical model is based on the denoised image, the renormalized-blurred image and the hot spot mask image.

The statistical model, in one embodiment, is based on a weighted average of the values of the pixels contained in the hot spot mask to generated $T_S$. In one embodiment, the weighted average corresponds to the arithmetic mean or median value of the values of the pixels in the hot spot mask to produce $T_S$.

The result of the weighted average may be further subjected to an empirical correction that arises from the size of the hot region and the estimated or measured distance between the thermal imaging camera and the subject of interest, as well as the ambient temperature. This empirical correction aims to eliminate errors due to atmospheric attenuation of thermal radiation, angle of viewing and background interference and the so-called size of source effect, which leads to an apparent attenuation of the temperature as an observed object of fixed size moves further away from the thermal imaging camera and vice versa. For example, the temperature apparently increases as the subject moves closer to the thermal imaging camera. This phenomenon may be exacerbated by the imperfection of the lens of the thermal sensor and the modulation transfer function of the lens. Additionally, the magnitude of the error induced by this effect increases when the ambient temperature is lowered in comparison to the subject's true skin temperature.

The $T_S$ determined by the skin temperature calculator 452 is processed by the core temperature calculator 456 to calculate Tc. In one embodiment, the core temperature calculator determines $T_C$ using an empirical biophysical model. The biophysical model is employed to infer core body temperatures based on the calculated skin temperature Ts and taking into account heat transfer properties, including ambient conditions which affect heat transfer properties, such as ambient temperature and humidity. For example, the biophysical model correlates the core body temperature Tc to the skin temperature $T_S$ based on ambient conditions or parameters, such as $T_A$ and $R_h$. The parameters of the biophysical model, for example, are stored in the storage unit. The storage unit may store other information required for calculating the core body temperature.

In one embodiment, the biophysical model is defined by a relationship of Tc to the calculated $T_S$ and current $T_A$ and $R_h$. In one embodiment, the biophysical model is defined as follows:

$$T_C = T_S + (T_S - T_A)\frac{h\lambda}{k} \qquad \text{(biophysical model equation)};$$

where

$T_C$ = the calculated core body temperature,
$T_S$ = the calculated skin temperature,
$T_A$ = the current ambient temperature,
h = the convection coefficient between the forehead skin and the ambient air,
$\lambda$ = a constant which is equal to a characteristic length,
k = the effective thermal conductivity of the layers between the brain from the outer surface of the forehead skin, and
$h\lambda/k$ = the parametric factor which defines the relationship between Tc, $T_S$ and $T_A$.

The parametric factor combines the standard physical quantities, h, k and $\lambda$. The parametric factor defines how quickly the brain temperature value, herein equated to Tc, is attenuated within the skull if the thermoregulatory process can maintain the brain temperature constant.

In one embodiment, extensive values for $h\lambda/k$ have been obtained through empirical tests correlating measured Tc, $T_S$, $T_A$ and $R_h$ in accordance to the biophysical model. The values are stored in the storage unit of the system. By knowing $T_S$, $T_A$ and $h\lambda/k$, Tc can be calculated.

Once Tc is calculated, it is passed to the output module 470. The output module is configured to inform the subject's core body temperature. For example, the Tc of the subject is displayed on a monitor (not shown). Should Tc exceed a threshold, such as indicating a fever, an alarm may be set. The alarm may be a visual alarm, an audio alarm or a combination thereof. Other types of alarms may also be useful. In some embodiments, the system is tied to a gate for allowing or blocking a subject from passing therethrough.

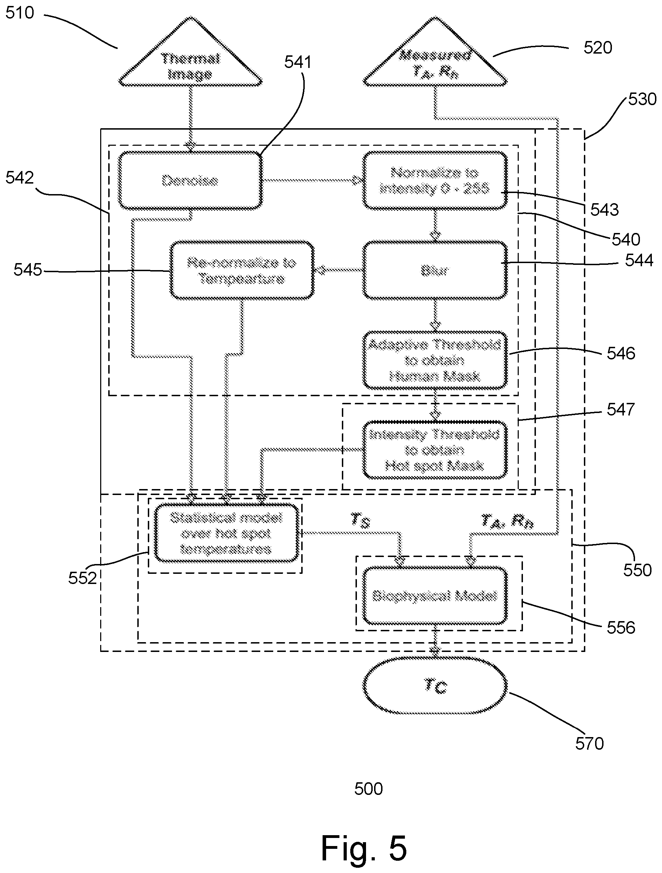

Fig. 5 shows a simplified flow diagram of another embodiment of a temperature screening process 500. As shown, the process may be performed by the system of Fig. 4. Common elements may not be described or described in detail.

As shown, the process includes simultaneous capturing of thermal images and ambient conditions at 510 and 520. For example, an input module with an image capture unit and an ambient sensor unit are configured to capture an image and ambient conditions simultaneously. The image capture unit may include a thermal imaging camera, such as a high-resolution multi-pixel CMOS-based long-wave infrared (LWIR) imaging camera. The image capture unit may be configured to capture images in a continuous, automatic or manual mode.

Each time an image is captured, ambient conditions are also captured. The ambient sensor unit may include sensors for capturing current interior ambient conditions, such as the ambient temperature $T_A$ and relative humidity $R_h$. The ambient sensor unit may include other sensors for measuring other ambient conditions. For example, the sensors may be configured to capture the temperature of the human subject's clothes and exterior ambient conditions, such as exterior temperature, humidity and air velocity, from where the human subjects came.

An image and its corresponding or current ambient conditions are processed. For example, the image and its current ambient conditions are processed by a processing module 530. The processing module, includes an image processing unit 540 and a temperature calculation unit 550.

In one embodiment, an incoming image is processed to detect a hot spot by the image processing unit. In one embodiment, the image processing unit generates a human mask. The human mask is generated by a human mask generator 542 of the image processing unit. The image is denoised at 541. For example, an image denoiser is employed to denoise the image to produce a denoised image. The image denoiser, for example, may be an AI-based image denoiser. The image denoiser generates a denoised image.

The denoised image, at 543, is normalized. For example, the denoised image is normalized by an image normalizer. Normalizing the image may include mapping the temperature values of the pixels of the image to integer values, such as from 0 - 255. The normalized image is blurred at 544. For example, blurring the normalized image may be performed using standard image processing techniques or a specialized AI model that helps to smoothen the boundaries of the contour of the human subject. In other words, blurring is the process of smoothening the edges of a human mask corresponding to a subject.

The blurred-normalized image is processed to generate a human mask at 546. The human mask is generated by the image mask generator using an adaptive thresholding process. The human mask, for example, is a segmented human subject image. The blurred-normalized image, in one embodiment, is also processed to renormalize the image due to blurring. For example, an image renormalizer renormalizes the blurred-normalized image to generate a renormalized-blurred image at 545.

The segmented human subject image is processed to generate a hot spot mask at 547. For example, a hot spot mask generator processes the segmented human subject image to generate a hot spot mask. In one embodiment, simple thresholding is employed to generate the hot spot mask. The hot spot mask, for example, is a segmented hot spot image of the segmented human subject image. The hot spot generally corresponds to the forehead of the human subject.

The process continues to process the outputs of the image processing unit to determine the Tc of the human subject. For example, the outputs of the image processing unit are processed by a temperature calculation unit 550 with a skin temperature calculator and a core temperature calculator.

In one embodiment, the denoised image, renormalized-blurred image and the segmented hot spot image are processed to determine a single skin temperature $T_S$ at 552. For example, the skin temperature calculator calculates $T_S$ based on the outputs from the image processing unit. In one embodiment, $T_S$ is calculated using a statistical model over the hot spot temperatures using a weighted average. In other embodiments, $T_S$ may be calculated using a dynamic weighted average.

At 556, $T_S$ is employed to generate Tc, which is estimated from the thermal image. Generating Tc is performed by the core temperature calculator. In one embodiment, Tc is generated using a biophysical model, taking Ts and ambient conditions, such as $T_A$ and $R_h$ into consideration. In one embodiment, the biophysical model may be defined by a relationship of Tc to the calculated $T_S$ and current $T_A$ and $R_h$. In one embodiment, the biophysical model is defined as follows:

$$T_C = T_S + (T_S - T_A)\frac{h\lambda}{k} \qquad \text{(biophysical model equation)}.$$

In one embodiment, extensive values for the parametric factor $h\lambda/k$ have been obtained through empirical tests correlating *a priori* measured Tc, $T_S$, $T_A$ and $R_h$ in accordance to the biophysical model. The values are stored in the storage unit of the system. By knowing $T_S$, $T_A$ and $h\lambda/k$, Tc can be calculated.

The core body temperature Tc is provided as an output at 570, completing the process for determining the Tc of the human subject from the image. Additional images may be processed or additional human subjects of the image may be processed.

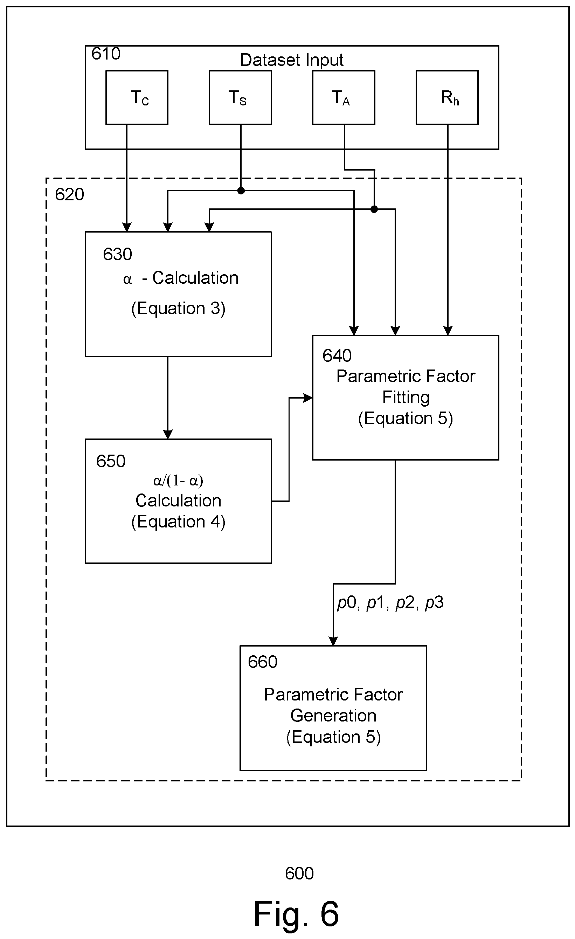

Fig. 6 shows an embodiment of a biophysical model 600. The biophysical model, for example, is employed by the core temperature calculator to calculate a core temperature Tc of a subject taking into account of current measured ambient conditions. In one embodiment, the biophysical model generates biophysical parameters as a function of $T_S$, $T_A$, $R_h$ and $\{p_i\}$, where $\{p_i\}$, for $i \in [0, 1, 2, 3]$, is a set of empirically established coefficients (constants) relating the parametric factor $h\lambda/k$ to the directly measured variables $T_S$, $T_A$ and $R_h$.

The biophysical model, as shown, includes an input module 610 and a processing module 620. The input of input unit 610 is a dataset of simultaneously measured Tc, $T_S$, $T_A$, and $R_h$. The processing module 620 includes an $\alpha$ calculation unit 630, a parametric factor fitting unit 640, an $\alpha/(1 - \alpha)$ calculation unit 650 and a parametric factor generation unit 660. Providing the biophysical model with other modules and units may also be useful.

In one embodiment, the input module 610 is configured to receive empirical data collected from actual measurements from subjects under various conditions. The dataset includes simultaneous measurements of parameters or variables. In one embodiment, the simultaneously measured parameters include Tc and $T_S$ of the subject as well as the ambient conditions $T_A$ and $R_h$. For example, the parameters Tc, $T_S$, $T_A$ and $R_h$ are measured at the same time. Furthermore, these parameters should be measured by the same operator and using the same instruments throughout the data collection process to avoid uncertainties and inconsistencies.

In one embodiment, the empirical dataset is analyzed by the processing module 620. The processing module, in one embodiment, is a biophysical model that estimates Tc by measuring $T_S$ and $T_A$. For example, the biophysical model is based on the biophysical model equation as follows:

$$T_C = T_S + (T_S - T_A)\frac{h\lambda}{k}$$ (biophysical model equation).

The biophysical model equation is derived from Equation 1 as follows:

$$T(r) = T_C - (T_C - T_A)\left(\frac{1}{1+\frac{k}{h\lambda}}\right)e^{-\frac{r}{\lambda}}$$ (Equation 1)

where,

$T(r)$ = the temperature at a position r from the skin surface in °C,
$r$ = the free variable in meters (m) which represents the depth from the forehead skin surface towards the center of the brain,
$\lambda$ = a characteristic length in m,
$k$ = thermal conductivity in W/m/K, and
$h$ = the heat exchange coefficient at the surface of the forehead in W/m$^2$/K.

Regarding h, it is generally dependent on the difference between skin and ambient temperature and air velocity, as well as other factors.

Equation 1 is derived from Penne's bioheat transfer equation. Equation 1 captures the influence of the environment through the boundary conditions. Equation 1 can be used to solve for $T_S$. The function T($r$) is solved with $r = 0$, which is at the forehead. The forehead skin temperature $T_S$ is normally different than Tc (e.g., the brain temperature) due to the ambient temperature $T_A$, leading to the heat exchange between the body and the environment. Solving Equation 1 using $r = 0$ results in the following Equation 2 expressing $T_S$ as a function of $T_C$ and $T_A$ as variables:

$$T_S = T(r = 0) = T_C - (T_C - T_A)\left(\frac{1}{1+\frac{k}{h\lambda}}\right)$$ (Equation 2).

where,

$k/h\lambda$ = the parametric factor.
Since r = 0, the exponential factor of Equation (1) evaluates to 1.

According to Equation 2, for a given ambient temperature $T_A$, the skin temperature $T_S$ is dependent on the core body temperature Tc. Tc is established and maintained by the active thermoregulatory systems of the body. In the case where only the relationship of Tc and the skin temperature $T_S$ is considered, the detailed temperature distribution inside the tissue as well as the exponential factor of Equation 1 can be ignored. Accordingly, based on Equation 2, with $T_A$, $T_S$ and Tc (or a proxy thereof, such as oral temperature) being known, the parametric factor $k/h\lambda$ can be determined. This will theoretically give us the relationship between the three different temperature values Tc, $T_S$ and $T_A$ for the entire human population.

In one embodiment, Equation 2 can be transformed into Equation 3 as follows:

$$\alpha = \frac{1}{1+k/(h\lambda)} = \frac{T_C-T_S}{T_C-T_A} \qquad \text{(Equation 3)},$$

where,

$\alpha$ = a coefficient representing the heat transfer state of the core body at a given ambient temperature.

Equation 3 enables the solving of the unknown parametric factor from a dataset of simultaneously measured temperatures Tc, $T_S$ and $T_A$.

In one embodiment, the $\alpha$ calculation unit 630 of the processing module determines $\alpha$ based on the measured Tc, $T_S$ and $T_A$ of the empirical dataset. For example, a linear least square fit may be used to determine $\alpha$. This minimizes the sum of the squared errors between a model function and data.

The calculated value of $\alpha$ based on Equation 3 is passed to the $\alpha/(1 - \alpha)$ calculation unit 650. The $\alpha/(1 - \alpha)$ calculation unit solves for the parametric factor $h\lambda/k$ using Equation 4 below:

$$\frac{h\lambda}{k} = \frac{\alpha}{1-\alpha} \qquad \text{(Equation 4)}.$$

By knowing the parametric factor $k/h\lambda$, the biophysical model equation could be solved to estimate Tc based on $T_S$, $T_A$ and $k/h\lambda$.

Since the parametric factor varies or is affected by the ambient conditions, including $R_h$, $\alpha/(1-\alpha)$ is passed to the parametric factor fitting unit 640. The parametric factor fitting model fits the parametric factor using the model Equation 5 below:

$$\frac{h\lambda}{k} = p_1 + p_1 T_S + p_2 T_A + p_3 R_h \qquad \text{(Equation 5)},$$

where,

$p0$, $p1$, $p2$, $p3$ = coefficients.

The coefficients $p0$, $p1$, $p2$, $p3$ are resulted from fitting the right-hand side of the model Equation 5 to the right-hand side of Equation 4, where the latter is obtained directly from the data points of the simultaneously measured Tc, $T_S$, $R_h$ and $T_A$ based on Equation 3. In other words, for every set of $T_C$, $T_S$ and $T_A$, a value for $\alpha$ is obtained using Equation 3. Then, the value of the right-hand side of Equation 4, namely $\alpha/(1 - \alpha)$ is calculated. To these resulting values, the coefficients $p0$, $p1$, $p2$, $p3$ are fitted using the right-hand side of Equation 5.

The parametric factor generation unit 660 generates the parameters $\{p_i\}$, which uniquely define the parametric factor $\frac{h\lambda}{k}$, according to Equation 5, for any set of measurable variables $T_S$, $T_A$, and $R_h$. The parameters, for example, are stored in the storage module. The parameters may be continuously updated by, for example, connecting to a centralized server. When a subject of an image is analyzed to determine his/her $T_S$, the parameters $p0$, $p1$, $p2$, $p3$ are used to obtain the parametric factor $\frac{h\lambda}{k}$ with the corresponding $T_A$ and $R_h$, and then to estimate the subject's Tc according to the biophysical equation.

The present disclosure may be embodied in other specific forms. The foregoing embodiments, therefore, are to be considered in all respects illustrative rather than limiting the invention described herein. The scope of the invention is thus indicated by the appended claims, rather than by the foregoing description.

**Claims**

1. A temperature scanning system comprising:

a thermal image capture module (410) configured to capture thermal images;
a storage unit;
an ambient sensor module (420) configured to capture ambient conditions including ambient temperature ($T_A$); and

a processing module (430) configured to process a thermal image, the processing module comprises an image processing unit (440), the image processing unit processes the thermal image and includes a human mask generator (442) configured to generate a human mask which extracts a first set of pixels representing the subject from the thermal image, and

a hot spot generator (447) configured to generate a hot spot mask which extracts a second set of pixels from the first set of pixels representing a forehead of the subject in the thermal image to detect a hot spot of the human subject in the thermal image; and

a temperature calculation unit (450), the temperature calculation unit is configured to

estimate a skin temperature (Ts) (452) of a subject in the thermal image, and
calculate a core temperature (Tc) (456) of the subject based on an arithmetic mean value or a median value of the pixels within the hot spot mask and current ambient conditions, using a biophysical model, wherein the biophysical model is defined as $Tc = T_S + (T_S - T_A) h\lambda / k$, wherein

Tc is the core temperature,

$T_S$ is the skin temperature as estimated by the temperature calculation unit,

$T_A$ is the current ambient temperature as determined by the ambient temperature sensor module,

h = the convection coefficient between the forehead skin and the ambient air,

$\lambda$ = a constant which is equal to a characteristic length,

k = the effective thermal conductivity of layers between the brain from the outer surface of the forehead skin, and

$h\lambda / k$ = the parametric factor which defines the relationship between Tc, $T_S$ and $T_A$; and the parameter being stored in the storage unit of the system (460).

2. The system of claim 1, wherein the ambient sensor module (420) comprises:

a temperature sensor for capturing an ambient temperature $T_A$ of an interior area proximate to the thermal image capture module; and
a humidity sensor for capturing a relative humidity $R_h$ of the interior area.

3. The system of any of claims 1-2 wherein the thermal image capture module comprises a LWIR high-resolution multi-pixel thermal imaging camera.

4. The system of any of claims 1-3 further comprises an output module (470) for generating a core temperature output for the Tc.

5. A method for non-contact temperature scanning comprising:

capturing a thermal image (210) of a subject with current ambient conditions;
processing the thermal image to detect a hot spot (230) of the subject, wherein the processing comprises

generating a subject mask (230) for the thermal image by extracting a first set of pixels representing the subject,
generating a hot spot mask for the hot spot on the subject mask by extracting a second set of pixels from the first set of pixels, and
determining a skin temperature $T_S$ (240) of the subject from the hot spot on the subject mask; and
calculating a core temperature Tc of the subject based on an arithmetic mean value or a median value of the pixels within the hot spot mask taking account of the current ambient conditions, which includes an ambient temperature ($T_A$) using a biophysical model, the biophysical model (260) is defined as $Tc = T_S + (T_S - T_A) h\lambda / k$, where

Tc is the core temperature,
$T_S$ is the skin temperature as estimated by the temperature calculation unit,
$T_A$ is the current ambient temperature,

h = the convection coefficient between the forehead skin and the ambient air,

$\lambda$ = a constant which is equal to a characteristic length,

k = the effective thermal conductivity of layers between the brain from the outer surface of the forehead skin, and

$h\lambda / k$ = the parametric factor which defines the relationship between Tc, $T_S$ and $T_A$, and

storing the parameter.

**6.** The method of claim 5 wherein:

$T_A$ is obtained by a temperature sensor located at an interior area proximate to a thermal image capture module for capturing the thermal image; and

a relative humidity $R_h$ of the interior area is captured by a humidity sensor.

**7.** The method of any of claims 5 and 6 capturing the thermal image comprises using a LWIR high-resolution multi-pixel thermal imaging camera.

**8.** The method of any of claims 5-7 further comprises outputting Tc by an output module (470).

**Patentansprüche**

**1.** Temperaturscansystem, umfassend:

ein Wärmebilderfassungsmodul (410), das dafür konfiguriert ist, Wärmebilder zu erfassen;
eine Speichereinheit;
ein Umgebungssensormodul (420), das dafür konfiguriert ist, Umgebungsbedingungen einschließlich der Umgebungstemperatur ($T_A$) zu erfassen; und
ein Verarbeitungsmodul (430), das dafür konfiguriert ist, ein Wärmebild zu verarbeiten, wobei das Verarbeitungsmodul Folgendes umfasst
eine Bildverarbeitungseinheit (440), wobei die Bildverarbeitungseinheit das Wärmebild verarbeitet und Folgendes beinhaltet
einen Menschenmaskengenerator (442), der dafür konfiguriert ist, eine Menschenmaske zu erzeugen, die einen ersten Satz von Pixeln, der das Subjekt darstellt, aus dem Wärmebild extrahiert, und
einen Hotspot-Generator (447), der dafür konfiguriert ist, eine Hotspot-Maske zu erzeugen, die aus dem ersten Satz von Pixeln einen zweiten Satz von Pixeln extrahiert, der eine Stirn des Subjekts im Wärmebild darstellt, um einen Hotspot des menschlichen Subjekts im Wärmebild zu erkennen; und
eine Temperaturberechnungseinheit (450), wobei die Temperaturberechnungseinheit für Folgendes konfiguriert ist Schätzen einer Hauttemperatur (Ts) (452) eines Subjekts in dem Wärmebild, und
Berechnen einer Kerntemperatur (Tc) (456) des Subjekts basierend auf einem arithmetischen Mittelwert oder einem Medianwert der Pixel innerhalb der Hotspot-Maske und aktuellen Umgebungsbedingungen unter Verwendung eines biophysikalischen Modells, wobei das biophysikalische Modell definiert ist als $T_C = T_S + (T_S - T_A)\, h\lambda / k$, wobei
$T_C$ die Kerntemperatur ist,
$T_S$ die von der Temperaturberechnungseinheit geschätzte Hauttemperatur ist,
$T_A$ die vom Umgebungstemperatursensormodul bestimmte aktuelle Umgebungstemperatur ist,
h = der Konvektionskoeffizient zwischen der Stirnhaut und der Umgebungsluft,
$\lambda$ = eine Konstante, die gleich einer charakteristischen Länge ist,
k = die effektive Wärmeleitfähigkeit von Schichten zwischen dem Gehirn und der Außenfläche der Stirnhaut, und
$h\lambda / k$ = der parametrische Faktor, der die Beziehung zwischen $T_C$, $T_S$ und $T_A$ definiert; und wobei der Parameter in der Speichereinheit des Systems (460) gespeichert wird.

**2.** System nach Anspruch 1, wobei das Umgebungssensormodul (420) Folgendes umfasst:

einen Temperatursensor zum Erfassen einer Umgebungstemperatur $T_A$ eines Innenbereichs in der Nähe des Wärmebilderfassungsmoduls; und
einen Feuchtigkeitssensor zum Erfassen einer relativen Feuchtigkeit $R_h$ des Innenbereichs.

**3.** System nach einem der Ansprüche 1-2, wobei das Wärmebilderfassungsmodul eine hochauflösende LWIR-Multipixel-Wärmebildkamera umfasst.

**4.** System nach einem der Ansprüche 1-3, das ferner ein Ausgabemodul (470) zum Erzeugen einer Kerntemperaturausgabe für die $T_C$ umfasst.

**5.** Verfahren zum kontaktlosen Temperaturscannen, umfassend:

Erfassen eines Wärmebildes (210) eines Subjekts bei aktuellen Umgebungsbedingungen;
Verarbeiten des Wärmebildes, um einen Hotspot (230) des Subjekts zu erkennen, wobei das Verarbeiten Folgendes umfasst
Erzeugen einer Subjektmaske (230) für das Wärmebild durch Extrahieren eines ersten Satzes von Pixeln, der das Subjekt darstellt,
Erzeugen einer Hotspot-Maske für den Hotspot auf der Subjektmaske durch Extrahieren eines zweiten Satzes von Pixeln aus dem ersten Satz von Pixeln, und
Bestimmen einer Hauttemperatur $T_S$ (240) des Subjekts anhand des Hotspots auf der Subjektmaske; und
Berechnen einer Kerntemperatur TC des Subjekts basierend auf einem arithmetischen Mittelwert oder einem Medianwert der Pixel innerhalb der Hotspot-Maske unter Berücksichtigung der aktuellen Umgebungsbedingungen, die eine Umgebungstemperatur ($T_A$) einschließen, unter Verwendung eines biophysikalischen Modells, wobei das biophysikalische Modell (260) definiert ist als $T_C = T_S + (T_S - T_A) h\lambda / k$, wobei
$T_C$ die Kerntemperatur ist,
$T_S$ die von der Temperaturberechnungseinheit geschätzte Hauttemperatur ist,
$T_A$ die aktuelle Umgebungstemperatur ist,
h = der Konvektionskoeffizient zwischen der Stirnhaut und der Umgebungsluft,
$\lambda$ = eine Konstante, die gleich einer charakteristischen Länge ist,
k = die effektive Wärmeleitfähigkeit von Schichten zwischen dem Gehirn und der Außenfläche der Stirnhaut, und
$h\lambda / k$ = der parametrische Faktor, der die Beziehung zwischen $T_C$, $T_S$ und $T_A$ definiert, und
Speichern des Parameters.

**6.** Verfahren nach Anspruch 5, wobei:

$T_A$ durch einen Temperatursensor erhalten wird, der sich in einem Innenbereich in der Nähe eines Wärmebilderfassungsmoduls zum Erfassen des Wärmebildes befindet; und
eine relative Feuchtigkeit $R_h$ des Innenbereichs durch einen Feuchtigkeitssensor erfasst wird.

**7.** Verfahren nach einem der Ansprüche 5 und 6, wobei das Erfassen des Wärmebildes die Verwendung einer hochauflösenden LWIR-Multipixel-Wärmebildkamera umfasst.

**8.** Verfahren nach einem der Ansprüche 5-7, das ferner das Ausgeben der $T_C$ durch ein Ausgabemodul (470) umfasst.

**Revendications**

**1.** Système de mesure de température comprenant :

un module de capture d'images thermiques (410) configuré pour capturer des images thermiques ;
une unité de stockage ;
un module de capteur ambiant (420) configuré pour capturer les conditions ambiantes, y compris la température ambiante ($T_A$) ; et
un module de traitement (430) configuré pour traiter une image thermique, le module de traitement comprend
une unité de traitement d'images (440), l'unité de traitement d'images traite l'image thermique et comporte
un générateur de masque humain (442) configuré pour générer un masque humain qui extrait un premier ensemble de pixels représentant le sujet à partir de l'image thermique, et
un générateur de points chauds (447) configuré pour générer un masque de points chauds qui extrait un second ensemble de pixels à partir du premier ensemble de pixels représentant le front du sujet dans l'image thermique afin de détecter un point chaud du sujet humain dans l'image thermique ; et
une unité de calcul de température (450), l'unité de calcul de température est configurée pour
estimer la température de la peau (Ts) (452) d'un sujet sur l'image thermique, et

calculer la température centrale (Tc) (456) du sujet à partir de la moyenne arithmétique ou de la médiane des pixels dans le masque de points chauds et des conditions ambiantes actuelles, en utilisant un modèle biophysique, dans lequel le modèle biophysique est défini comme $T_C = T_S + (T_S - T_A) h\lambda/k$, dans lequel $T_C$ est la température centrale,

$T_S$ est la température de la peau estimée par l'unité de calcul de température,

$T_A$ est la température ambiante actuelle telle que déterminée par le module de capteur de température ambiante,

h = le coefficient de convection entre la peau du front et l'air ambiant,

$\lambda$ = une constante égale à une longueur caractéristique,

k = la conductivité thermique effective de couches situées entre le cerveau et la surface externe de la peau du front, et

$h\lambda/k$ = le facteur paramétrique qui définit la relation entre $T_C$, $T_S$ et $T_A$ ; et le paramètre étant stocké dans l'unité de stockage du système (460).

2. Système selon la revendication 1, dans lequel le module de capteur ambiant (420) comprend :

un capteur de température pour capturer la température ambiante $T_A$ d'une zone intérieure proche du module de capture d'images thermiques ; et

un capteur d'humidité pour capturer l'humidité relative $R_h$ de la zone intérieure.

3. Système selon l'une quelconque des revendications 1 et 2, dans lequel le module de capture d'images thermiques comprend une caméra d'imagerie thermique multipixel haute résolution LWIR.

4. Système selon l'une quelconque des revendications 1 à 3, qui comprend en outre un module de sortie (470) pour générer une sortie de température centrale pour la $T_C$.

5. Procédé de mesure de température sans contact comprenant :

la capture d'une image thermique (210) d'un sujet avec les conditions ambiantes actuelles ;

le traitement de l'image thermique pour détecter un point chaud (230) du sujet, dans lequel le traitement comprend

la génération d'un masque de sujet (230) pour l'image thermique en extrayant un premier ensemble de pixels représentant le sujet,

la génération d'un masque de point chauds pour le point chaud sur le masque de sujet en extrayant un second ensemble de pixels à partir du premier ensemble de pixels, et

la détermination d'une température de la peau $T_S$ (240) du sujet à partir du point chaud sur le masque de sujet ; et

le calcul de la température centrale Tc du sujet à partir de la moyenne arithmétique ou de la médiane des pixels dans le masque de points chauds en prenant en compte les conditions ambiantes actuelles, qui comporte une température ambiante ($T_A$), en utilisant un modèle biophysique, le modèle biophysique (260) est défini comme $T_C = T_S + (T_S - T_A) h\lambda/k$, où

$T_C$ est la température centrale,

$T_S$ est la température de la peau estimée par l'unité de calcul de température,

$T_A$ est la température ambiante actuelle,

h = le coefficient de convection entre la peau du front et l'air ambiant,

$\lambda$ = une constante égale à une longueur caractéristique,

k = la conductivité thermique effective de couches situées entre le cerveau et la surface externe de la peau du front, et

$h\lambda/k$ = le facteur paramétrique qui définit la relation entre $T_C$, $T_S$ et $T_A$, et

le stockage du paramètre.

6. Procédé selon la revendication 5, dans lequel :

$T_A$ est obtenue grâce à un capteur de température situé dans une zone intérieure proche d'un module de capture d'images thermiques pour capturer l'image thermique ; et

l'humidité relative $R_h$ de la zone intérieure est capturée par un capteur d'humidité.

7. Procédé selon l'une quelconque des revendications 5 et 6, de capture de l'image thermique qui comprend l'utilisation d'une caméra d'imagerie thermique multipixel haute résolution LWIR.

8. Procédé selon l'une quelconque des revendications 5 à 7, qui comprend en outre la sortie de $T_C$ par un module de sortie (470).

110 Image Capture Module

120 Ambient Sensor Module

130 Processing Module

140 Image Processing Unit

160 Storage Unit

150 Temperature Calculation Unit

152 Skin Temperature Calculator

156 Core Temperature Calculator

170 Output Module

100

Fig. 1

Fig. 2

Fig. 3a

Fig. 3b

410
Image
Capture
Module

420
Ambient
Sensor Module

430 Processing Module

440 Image Processing Unit

442
Human Mask
generator

447
Hot Spot
Mask Generator

460
Storage
Unit

450 Temperature Calculation
Unit

452
Skin Temperature
Calculator

456
Core Temperature
Calculator

470 Output
Module

400

Fig. 4

510

541

520

530

542

543

540

544

545

546

547

552

550

556

570

Thermal Image

Measured $T_A$, $R_A$

Denoise

Normalize to intensity 0 - 255

Re-normalize to Tempearture

Blur

Adaptive Threshold to obtain Human Mask

Intensity Threshold to obtain Hot spot Mask

Statistical model over hot spot temperatures

$T_S$

$T_A$, $R_A$

Biophysical Model

$T_C$

500

# Fig. 5

600

# Fig. 6

**EP 4 182 650 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008154138 A1
- CN 110196103 A